# EUROPEAN PATENT APPLICATION

(11) **EP 2 782 068 A2**
(43) Date of publication of application: **24.09.2014**
(21) Application number: 14160987.5
(22) Date of filing: 21.03.2014
(51) Int. Cl.: G06T 7/00

(54) **Apparatus and method for providing elasticity information**

(30) Priority: 22.03.2013 KR 20130031111; 17.09.2013 KR 20130112064
(71) Applicant: Samsung Medison Co., Ltd., Gangwon-do 250-870 (KR)
(72) Inventor: Shin, Dong-kuk, Gangwon-do (KR); Choe, Seong-hyeon, Gangwon-do (KR); Kim, Jong-sik, Gangwon-do (KR)
(74) Representative: Schmid, Wolfgang

(57) **Abstract**

Provided is a method for providing elasticity information. The method includes: acquiring an elasticity image of an object; classifying the elasticity image into at least one similar tissue; determining a reference strain based on a strain of a reference similar tissue among the at least one similar tissue; and outputting a ratio or a difference between the reference strain and a strain of an interesting region of the elasticity image.

## Description

### RELATED APPLICATIONS

This application claims the benefit of Korean Patent Application No. 10-2013-0031111, filed on March 22, 2013, and Korean Patent Application No. 10-2013-0112064, filed on September 17, 2013, in the Korean Intellectual Property Office, the disclosures of which are incorporated herein in their entirety by reference.

### BACKGROUND

### 1. Field

One or more embodiments of the present invention relate to apparatuses and methods for providing elasticity information, and more particularly, to apparatuses and methods for providing a user with elasticity information that is acquired from an elasticity image of an object.

### 2. Description of the Related Art

An ultrasound system is an equipment for observing an internal structure of an organism. The ultrasound system is a noninvasive test apparatus, and shows structural details in a physical body, internal tissues, and a fluid flow.

The ultrasound system transmits ultrasound signals to an object and generates an ultrasound image of the object by using response signals that are reflected from the object.

The ultrasound image is mainly represented as a brightness (B) mode image based on a reflection coefficient caused by an impedance difference between tissues. However, a portion such as a malignant tumor, which has a smaller reflection coefficient difference than surrounding tissues, is hardly observed in the B mode image. A malignant tumor or the like included in the object may be observed in an elasticity image that visualizes mechanical characteristics of a tissue. In the B mode image, it is difficult to discriminate between a normal tissue and an abnormal tissue since a scattering efficiency difference between the normal tissue and the abnormal tissue is not great. However, in the elasticity image generated by using a mechanical reaction difference of a medium between a case where external pressure is applied thereto and a case where no external pressure is applied thereto, it is possible to discriminate between a normal tissue and an abnormal tissue.

The elasticity image greatly assists in diagnosing a disease since it visualizes mechanical characteristics of tissues that may not be diagnosed in the B mode image.

Since a malignant tumor is harder than surrounding soft tissues, the malignant tumor is less deformed than the surrounding soft tissues, when the same external pressure is applied thereto. Thus, a user may identify a disease such as a malignant tumor from the elasticity image.

A degree of deformation of the object by pressure applied per unit area is referred to as a strain. In an related art method, when a user desires to know elasticity information or a strain of a predetermined region of an elasticity image, an ultrasound system receives a selection of a reference region from the user and provides the user with a difference between a strain of the reference region and a strain of the predetermined region.

According to the related art method, when the reference region selected by the user is harder than a normal tissue, a disease such as a malignant tumor, which has a small strain difference from the strain of the reference region, may also be determined as a normal tissue. Also, since the elasticity information of the predetermined region may vary according to users selecting the reference region, the reliability of the elasticity information may be reduced.

### SUMMARY

One or more embodiments of the present invention include apparatuses and methods for providing a user with reliable elasticity information of an interesting region of an object.

One or more embodiments of the present invention include apparatuses and methods that allow a user to easily identify a disease from an elasticity image of an object.

Additional aspects will be set forth in part in the description which follows and, in part, will be apparent from the description, or may be learned by practice of the presented embodiments.

According to one or more embodiments of the present invention, a method for providing elasticity information includes: acquiring an elasticity image of an object; classifying the elasticity image into at least one similar tissue; determining a reference strain based on a strain of a reference similar tissue among the at least one similar tissue; and outputting a ratio or a difference between the reference strain and a strain of an interesting region of the elasticity image that is input by a user.

The method may further include mapping a ratio or a difference between the reference strain and a strain of each of a plurality of regions included in the elasticity image, to a color scale, and displaying a color corresponding to each of the plurality of regions in each of the plurality of regions.

The color scale may include a lightness scale or a chroma scale of a first color.

The displaying of the color corresponding to each of the plurality of regions in each of the plurality of regions may include displaying a region among the plurality of regions, a ratio or a difference between the reference strain and a strain of which is greater than a predetermined reference value, in a first color, and displaying a region among the plurality of regions, a ratio or a difference between the reference strain and a strain of which is smaller than or equal to the predetermined reference value, in a second color that is different from the first color.

The method may further include: receiving a selection of a region included in the elasticity image, from the user; changing the reference strain according to a strain of the region selected by the user; and mapping a ratio or a difference between the reference strain and a strain of each of a plurality of regions included in the elasticity image, to a color scale, and displaying a color corresponding to each of the plurality of regions in each of the plurality of regions.

The classifying of the elasticity image into at least one similar tissue may include classifying the elasticity image into at least one similar tissue based on at least one of an attenuation coefficient value, a phase value, and a intensity value of each of a plurality of first response signals that are reflected from the object while no pressure is applied to the object.

The classifying of the elasticity image into at least one similar tissue may include classifying the elasticity image into at least one similar tissue based on at least one of an attenuation coefficient value, a phase value, and a intensity value of each of a plurality of second response signals that are reflected from the object while pressure is applied to the object.

The classifying of the elasticity image into at least one similar tissue may include classifying the elasticity image into at least one similar tissue based on at least one of a texture of the elasticity image, a geometry of the elasticity image, and a gradient of the elasticity image.

The acquiring of the elasticity image may include acquiring the elasticity image that is generated based on a strain between first ultrasound data of the object, which is acquired while pressure is not applied to the object, and second ultrasound data of the object, which is acquired while pressure is applied to the object.

The classifying of the elasticity image into at least one similar tissue may include: classifying a brightness (B) mode image of the object, which is generated by using the first ultrasound data or the second ultrasound data, into at least one similar tissue; and classifying the elasticity image into at least one similar tissue based on location information of each of the at least one similar tissue of the B mode image.

The classifying of the elasticity image into at least one similar tissue may include determining a region, which emits a first response signal having at least one of an attenuation coefficient value within a predetermined attenuation coefficient range, a phase value within a predetermined phase range, and a intensity value within a predetermined intensity range among a plurality of first response signals that are reflected from the object while no pressure is applied to the object, as the reference similar tissue.

The classifying of the elasticity image into at least one similar tissue may include determining a region, which emits a second response signal having at least one of an attenuation coefficient value within a predetermined attenuation coefficient range, a phase value within a predetermined phase range, and a intensity value within a predetermined intensity range among a plurality of second response signals that are reflected from the object while pressure is applied to the object, as the reference similar tissue.

The determining of the reference strain based on the strain of the reference similar tissue among the at least one similar tissue may include determining the reference strain based on a frequency value, a median value, or an average value of a plurality of strains that are acquired from the reference similar tissue.

The at least one similar tissue may include at least one of a skin tissue, a fat tissue, a mammary gland tissue, a muscle tissue, and a skeleton tissue of the object.

The reference similar tissue may include a fat tissue of the object.

According to one or more embodiments of the present invention, a method for providing elasticity information includes: acquiring an elasticity image of an object; classifying the elasticity image into at least one similar tissue; determining a reference strain based on a strain of a reference similar tissue among the at least one similar tissue; and mapping a ratio or a difference between the reference strain and a strain of each of a plurality of regions included in the elasticity image, to a color scale, and displaying a color corresponding to each of the plurality of regions in each of the plurality of regions.

A computer program for executing the elasticity information providing method may be recorded in a computer-readable recording medium.

According to one or more embodiments of the present invention, a method for providing elasticity information includes: acquiring an elasticity image of an object; classifying the elasticity image into at least one similar tissue; determining a reference strain based on a strain of a reference similar tissue among the at least one similar tissue; and outputting a ratio or a difference between the reference strain and a strain of an interesting region of the elasticity image that is input by a user.

According to one or more embodiments of the present invention, an apparatus for providing elasticity information, includes: an elasticity image acquiring unit configured to acquire an elasticity image of an object; a similar tissue classifying unit configured to classify the elasticity image into at least one similar tissue; a strain determining unit configured to determine a reference strain based on a strain of a reference similar tissue among the at least one similar tissue; and an output unit configured to output a ratio or a difference between the reference strain and a strain of an interesting region of the elasticity image.

The output unit may map a ratio or a difference between the reference strain and a strain of each of a plurality of regions included in the elasticity image, to a color scale, and display a color corresponding to each of the plurality of regions in each of the plurality of regions.

The color scale may include a lightness scale or a chroma scale of a first color.

The display may display a region among the plurality of regions, a ratio or a difference between the reference strain and a strain of which is greater than a predetermined reference value, in a first color, and display a region among the plurality of regions, a ratio or a difference between the reference strain and a strain of which is smaller than or equal to the predetermined reference value, in a second color that is different from the first color.

The apparatus may further include a user input unit configured to receive a selection of a region included in the elasticity image, from the user, wherein the strain determining unit may change the reference strain according to a strain of the region selected by the user, and the display may map a ratio or a difference between the reference strain and a strain of each of a plurality of regions included in the elasticity image, to a color scale, and display a color corresponding to each of the plurality of regions in each of the plurality of regions.

The similar tissue classifying unit may classify the elasticity image into at least one similar tissue based on at least one of an attenuation coefficient value, a phase value, and a intensity value of each of a plurality of first response signals that are reflected from the object while no pressure is applied to the object.

The similar tissue classifying unit may classify the elasticity image into at least one similar tissue based on at least one of an attenuation coefficient value, a phase value, and a intensity value of each of a plurality of second response signals that are reflected from the object while pressure is applied to the object.

The similar tissue classifying unit may classify the elasticity image into at least one similar tissue based on at least one of a texture of the elasticity image, a geometry of the elasticity image, and a gradient of the elasticity image.

The elasticity image of the object may be generated based on a strain between first ultrasound data of the object, which is acquired while pressure is not applied to the object, and second ultrasound data of the object, which is acquired while pressure is applied to the object.

The similar tissue classifying unit may classify a brightness (B) mode image of the object, which is generated by using the first ultrasound data or the second ultrasound data, into at least one similar tissue, and classify the elasticity image into at least one similar tissue based on location information of each of the at least one similar tissue of the B mode image.

The apparatus may further include a reference similar tissue determining unit configured to determine a region, which emits a first response signal having at least one of an attenuation coefficient value within a predetermined attenuation coefficient range, a phase value within a predetermined phase range, and a intensity value within a predetermined intensity range among a plurality of first response signals that are reflected from the object while no pressure is applied to the object, as the reference similar tissue.

The apparatus may further include a reference similar tissue determining unit configured to determine a region, which emits a second response signal having at least one of an attenuation coefficient value within a predetermined attenuation coefficient range, a phase value within a predetermined phase range, and a intensity value within a predetermined intensity range among a plurality of second response signals that are reflected from the object while pressure is applied to the object, as the reference similar tissue.

The strain determining unit may determine the reference strain based on a frequency value, a median value, or an average value of a plurality of strains that are acquired from the reference similar tissue.

The at least one similar tissue may include at least one of a skin tissue, a fat tissue, a mammary gland tissue, a muscle tissue, and a skeleton tissue of the object.

The reference similar tissue may include a fat tissue of the object.

According to one or more embodiments of the present invention, an apparatus for providing elasticity information includes: an elasticity image acquiring unit configured to acquire an elasticity image of an object; a similar tissue classifying unit configured to classify the elasticity image into at least one similar tissue; a strain determining unit configured to determine a reference strain based on a strain of a reference similar tissue among the at least one similar tissue; and an output unit configured to map a ratio or a difference between the reference strain and a strain of each of a plurality of regions included in the elasticity image, to a color scale, and display a color corresponding to each of the plurality of regions in each of the plurality of regions.

### BRIEF DESCRIPTION OF THE DRAWINGS

These and/or other aspects will become apparent and more readily appreciated from the following description of the embodiments, taken in conjunction with the accompanying drawings in which:
FIG. 1 is a diagram illustrating a general method for acquiring an elasticity image of an object;
FIG. 2 is a block diagram illustrating an elasticity information providing apparatus according to an embodiment of the present invention;
FIG. 3A is a diagram illustrating an elasticity image that is classified into at least one similar tissue;
FIG. 3B is a diagram illustrating a brightness (B) mode image that is classified into at least one similar tissue;
FIG. 4 is a diagram illustrating elasticity information of an interesting region that is output by an output unit;
FIGS. 5A and 5B are diagrams illustrating an elasticity image according to an embodiment of the present invention;
FIG. 6 is a block diagram illustrating an elasticity information providing apparatus according to another embodiment of the present invention;
FIG. 7 is a block diagram illustrating a wireless probe that may be connected to the elasticity information providing apparatus illustrated in FIG. 6;
FIG. 8 is a flowchart illustrating an elasticity information providing method according to another embodiment of the present invention; and
FIG. 9 is a flowchart illustrating an elasticity information providing method according to another embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, expressions such as "at least one of," when preceding a list of elements, modify the entire list of elements and do not modify the individual elements of the list.

The effects and features of the present invention and the accomplishing method thereof will become apparent from the following description of the embodiments, taken in conjunction with the accompanying drawings. The invention may, however, be embodied in many different forms and should not be construed as being limited to the embodiments set forth herein; rather, these embodiments are provided such that this disclosure will be thorough and complete, and will fully convey the concept of the invention to one of ordinary skill in the art. Like reference numerals refer to like elements throughout the specification.

The term "unit" used herein refers to a software component or a hardware component such as a field-programmable gate array (FPGA) or an application-specific integrated circuit (ASIC), and the "unit" performs some functions. However, the "unit" is not limited to software or hardware. The "unit" may be formed so as to be in a addressable storage medium, or may be formed so as to operate one or more processors. Thus, for example, the "unit" may include components such as software components, object-oriented software components, class components, and task components, and may include processes, functions, attributes, procedures, subroutines, segments of program code, drivers, firmware, micro codes, circuits, data, a database, data structures, tables, arrays, and variables. A function provided by the components and "units" may be associated with the smaller number of components and "units", or may be divided into additional components and "units".

In the specification, an "image" may mean multi-dimensional data formed of discrete image elements (e.g., pixels in a two-dimensional (2D) image and voxels in a three-dimensional (3D) image). For example, the image may include a medical image of a object that is acquired by a computed tomography (CT) apparatus, a magnetic resonance imaging (MRI) apparatus, an ultrasound apparatus, or other medical image apparatuses

Also, an "object" may include a human, an animal, or a part of a human or animal. For example, the object may include organs such as liver, heart, womb, brain, breast, abdomen, or the like, or a blood vessel. Also, the object may include a phantom. The phantom may refer to a material having a volume that is very close to a density and effective atomic number of an organism, and may include a spherical phantom having a characteristic similar to a physical body.

Also, a "user" may be, but is not limited to, a medical expert including a doctor, a nurse, a medical laboratory technologist, a medial image expert, and a technician who repairs a medical apparatus.

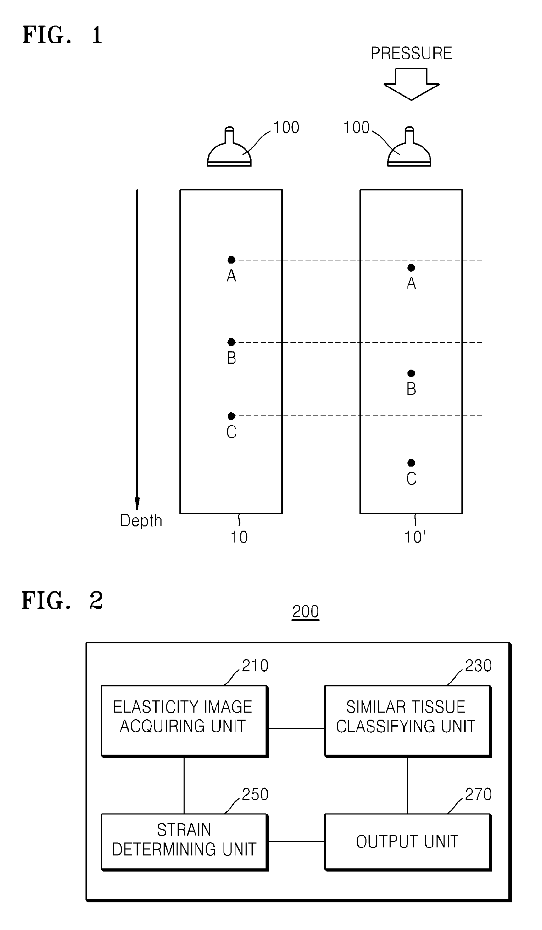

FIG. 1 is a diagram illustrating a general method for acquiring an elasticity image of an object.

A probe 100 of an ultrasound system transmits an ultrasound signal to an object 10 to which no pressure is applied, and receives a plurality of first response signals that are reflected from reflection points A, B, and C. The probe 100 acquires first ultrasound data corresponding to frame data by using the plurality of first response signals. The first ultrasound data may be generated based on at least one of an attenuation coefficient value, a phase value, and a intensity value of each of the plurality of first response signals. Thereafter, the probe 100 transmits an ultrasound signal to an object 10' to which pressure is applied, and receives a plurality of second response signals that are reflected from reflection points A, B, and C. The probe 100 acquires second ultrasound data corresponding to frame data by using the plurality of second response signals. The second ultrasound data may be generated based on at least one of an attenuation coefficient value, a phase value, and a intensity value of each of the plurality of second response signals.

Referring to FIG. 1, it may be seen that the positions of the reflection points A, B, and C of the object 10' to which pressure is applied are different from the positions of the reflection points A, B, and C of the object 10 to which no pressure is applied. That is, when pressure is applied to the object 10, the positions of the reflection points A, B, and C change according to a direction in which the pressure is applied. A position change degree of the reflection points A, B, and C increases as a distance from the probe 100 increases.

Due to a position difference between the reflection points A, B, and C of the object 10' to which pressure is applied and the reflection points A, B, and C of the object 10 to which pressure is not applied, the second ultrasound data is delayed in comparison with the first ultrasound data. Thus, the displacement of tissues in the object may be calculated by calculating the delay degree of the second ultrasound data. When a gradient is calculated by differentiating a displacement function, a strain value of each tissue of the object is acquired. An elasticity image may be generated based on the strain value. The displacement of the tissues in the object may be calculated by cross-correlation or autocorrelation.

FIG. 2 is a block diagram illustrating an elasticity information providing apparatus 200 according to an embodiment of the present invention.

Referring to FIG. 2, the elasticity information providing apparatus 200 according to an embodiment of the present invention may include an elasticity image acquiring unit 210, a similar tissue classifying unit 230, a strain determining unit 250, and an output unit 270. The elasticity image acquiring unit 210, the similar tissue classifying unit 230, and the strain determining unit 250 may be configured by a microprocessor.

The elasticity image acquiring unit 210 acquires an elasticity image of an object. The elasticity image may be generated based on a strain between first ultrasound data of an object that is acquired while pressure is not applied to the object and second ultrasound data of the object that is acquired while pressure is applied to the object.

The elasticity image acquiring unit 210 may acquire an elasticity image of the object from an external server or an external medical image photographing apparatus. Also, the elasticity image acquiring unit 210 may acquire an elasticity image of the object by photographing an ultrasound image of the object.

The similar tissue classifying unit 230 classifies the elasticity image, which is acquired by the elasticity image acquiring unit 210, into at least one similar tissue. The similar tissue may refer to a set of cells that have similar shapes and functions. The at least one similar tissue may include at least one of a skin tissue, a fat tissue, a mammary gland tissue, a muscle tissue, and a skeleton tissue of the object.

The similar tissue classifying unit 230 may classify all regions of the elasticity image into at least one similar tissue, or may classify some regions of the elasticity image into at least one similar tissue. In general, since most diseases such as tumors are distributed in mammary gland tissues, a user desires to identify whether a disease such as a tumor is included in a mammary gland tissue of the elasticity image. Thus, when an interesting region is set, the similar tissue classifying unit 230 may determine some regions to be classified into at least one similar tissue, in consideration of the position of the interesting region. Herein, the interesting region may be set by the user as ROI Region of Interest. For example, when the user desires to determine a fat tissue of an object as a reference similar tissue, since a fat tissue is located between a mammary gland tissue and a skin tissue, some regions except a region from an interesting region selected by the user to a skeleton tissue among all regions of the elasticity image into at least one similar tissue. Accordingly, a process complexity and a process time taken to classify the elasticity image into at least one similar tissue may be reduced.

The similar tissue classifying unit 230 may classify the elasticity image into at least one similar tissue by various methods.

As a first example, the similar tissue classifying unit 230 may classify the elasticity image into at least one similar tissue based on at least one of an attenuation coefficient value, a phase value, and a intensity value of each of a plurality of first response signals that are reflected from the object while no pressure is applied to the object.

Also, the similar tissue classifying unit 230 may classify the elasticity image into at least one similar tissue based on at least one of an attenuation coefficient value, a phase value, and a intensity value of each of a plurality of second response signals that are reflected from the object while pressure is applied to the object.

Since response signals reflected from cells having similar shapes and functions have similar characteristics, the similar tissue classifying unit 230 may group a plurality of first response signals or a plurality of second response signals according to the characteristics of response signals and classify the elasticity image into at least one similar tissue.

As a second example, the similar tissue classifying unit 230 may classify the elasticity image into at least one similar tissue based on at least one of a texture of the elasticity image, a geometry of the elasticity image, and a gradient of the elasticity image. That is, the similar tissue classifying unit 230 may classify the elasticity image into regions having similar characteristics, based on the image characteristics of the elasticity image.

As a third example, the similar tissue classifying unit 230 may classify a brightness (B) mode image of the object, which is generated from first ultrasound data or second ultrasound data, into at least one similar tissue, and classify the elasticity image into at least one similar tissue based on location information of each of the at least one similar tissue of the B mode image. For example, the similar tissue classifying unit 230 may classify the B mode image into at least one similar tissue based on at least one of a texture of the B mode image, a geometry of the B mode image, and a gradient of the B mode image.

The strain determining unit 250 determines a reference strain based on a stain of a reference similar tissue among at least one similar tissue of the elasticity image. In general, since a fat tissue of a physical body has a constant elasticity and a disease such as a malignant tumor is scarcely generated in the fat tissue, the strain determining unit 250 may determine a reference strain based on a strain of a fat tissue of the object. According to embodiments, the strain determining unit 250 may determine a reference strain based on a strain of a mammary gland tissue, a muscle tissue, or the like other than the fat tissue of the object.

A plurality of different strains may be acquired from the reference similar tissue, since even cells included in one similar tissue may have different respective characteristics. Thus, when a plurality of strains are acquired from the reference similar tissue, the strain determining unit 250 may determine a reference strain based on a frequency value, a median value, or an average value of the acquired strains. In detail, the strain determining unit 250 may determine an average value or a median value of a plurality of strains, which are acquired from the reference similar tissue, as a reference strain, or may determine a strain having the highest frequency among the plurality of strains as a reference strain.

The output unit 270 may output a ratio or a difference between the reference strain and a strain of an interesting region of the elasticity image. The interesting region of the elasticity image may be set by the user as region of interest ROI. The output unit 270 may include a display, a speaker, a printer, and the like that provide information, and may include various other output devices that are known to those of ordinary skill in the art.

The ratio between the reference strain and the strain of the interesting region input by the user may include the ratio of the reference strain to the strain of the interesting region (the reference strain ÷ the strain of the interesting region) and the ratio of the strain of the interesting region to the reference strain (the strain of the interesting region ÷ the reference strain). The difference between the reference strain and the strain of the interesting region may include the difference of the reference strain from the strain of the interesting region (the strain of the interesting region - the reference strain) and the difference of the strain of the interesting region from the reference strain (the reference strain - the strain of the interesting region).

The elasticity information providing apparatus 200 may further include a reference similar tissue determining unit (not illustrated).

The reference similar tissue determining unit may determine a reference similar tissue among at least one similar tissue classified from the elasticity image. The reference similar tissue determining unit may determine a reference similar tissue by various methods.

As a first example, the reference similar tissue determining unit may determine a region, which emits a first response signal having at least one of an attenuation coefficient value within a predetermined attenuation coefficient range, a phase value within a predetermined phase range, and a signal intensity value within a predetermined intensity range among a plurality of first response signals that are reflected from the object while pressure is not applied to the object, as a reference similar tissue.

Also, the reference similar tissue determining unit may determine a region, which emits a second response signal having at least one of an attenuation coefficient value within a predetermined attenuation coefficient range, a phase value within a predetermined phase range, and a signal intensity value within a predetermined intensity range among a plurality of second response signals that are reflected from the object while pressure is applied to the object, as a reference similar tissue.

The predetermined signal intensity range, the predetermined phase range, and the predetermined attenuation coefficient range may be preset by the user. In detail, when the user desires to determine a fat tissue as a reference similar tissue, the user may predetermine an attenuation coefficient range, a phase range, and a signal intensity range of a response signal that is emitted from an average fat tissue. The reference similar tissue determining unit may determine a region, which emits a first response signal having at least one of an attenuation coefficient value within a predetermined attenuation coefficient range set by the user, a phase value within a predetermined phase range set by the user, and a signal intensity value within a predetermined intensity range set by the user among a plurality of first response signals, as a fat tissue.

As a second example, the reference similar tissue determining unit may determine a reference similar tissue among at least one similar tissue by using at least one of a texture of the elasticity image or the B mode image, a geometry of the elasticity image, and a gradient of the elasticity image. In detail, the reference similar tissue determining unit may determine a reference similar tissue from at least one similar tissue classified from the elasticity image, by comparing at least one of a texture, a geometry, and a gradient of the elasticity image or the B mode image of the average fat tissue and at least one of a texture, a geometry, and a gradient of the elasticity image or the B mode image of the object.

The elasticity information providing apparatus 200 may automatically determine a reference strain of the reference similar tissue, even without receiving a selection of a reference region from the user. That is, the user may identify elasticity information of an interesting region just by selecting the interesting region ROI from the elasticity image. Also, since the elasticity information providing apparatus 200 automatically determines a strain of the reference similar tissue of the object as a reference strain, elasticity information of an interesting region does not vary according to users.

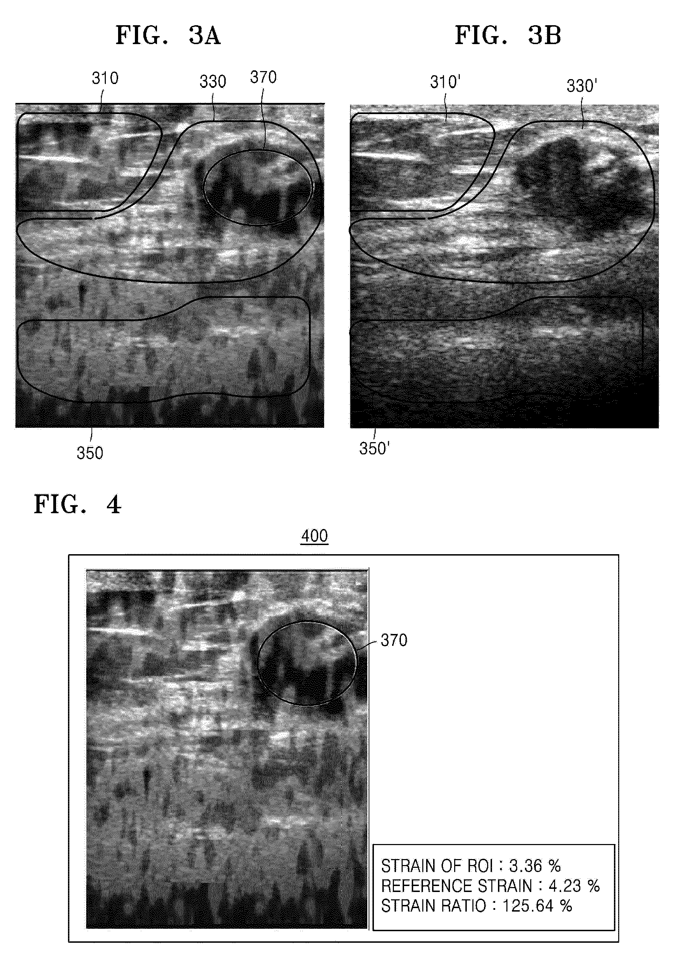

FIG. 3A is a diagram illustrating an elasticity image that is classified into at least one similar tissue.

Referring to FIG. 3A, an elasticity image may be classified into three similar tissues 310, 330, and 350. A reference numeral "370" denotes an interesting region. In detail, the interesting region 370 is selected by the user as a ROI.

In FIG. 3A, the first similar tissue 310 may correspond to a fat tissue, the second similar tissue 330 may correspond to a mammary gland tissue, and the third similar tissue 350 may correspond to a muscle tissue.

FIG. 3B is a diagram illustrating a B mode image that is classified into at least one similar tissue.

Referring to FIG. 3B, a B mode image may be classified into three regions 310', 330', and 350' like the elasticity image of FIG. 3A.

The similar tissue classifying unit 230 may classify the elasticity image into a first similar tissue 310, a second similar tissue 330, and a third similar tissue 350 based on location information of a first similar tissue 310', a second similar tissue 330', and a third similar tissue 350' of the B mode image. Accordingly, the first similar tissue 310, the second similar tissue 330, and the third similar tissue 350 of the elasticity image may be classified more accurately.

FIG. 4 is a diagram illustrating elasticity information of an interesting region that is output by the output unit 270. The output unit 270 may include a display 400 that displays elasticity information.

As illustrated in FIG. 4, when receiving a selection of an interesting region 370 included in an elasticity image from the user, the display 400 may display a screen including information comprising a reference strain as "REFERENCE STRAIN: 4.23%", a strain of the interesting region 370 as "STRAIN OF ROI: 3.36%", and a ratio between the reference strain and the strain of the interesting region 370 as "STRAIN RATIO: 125.64%".

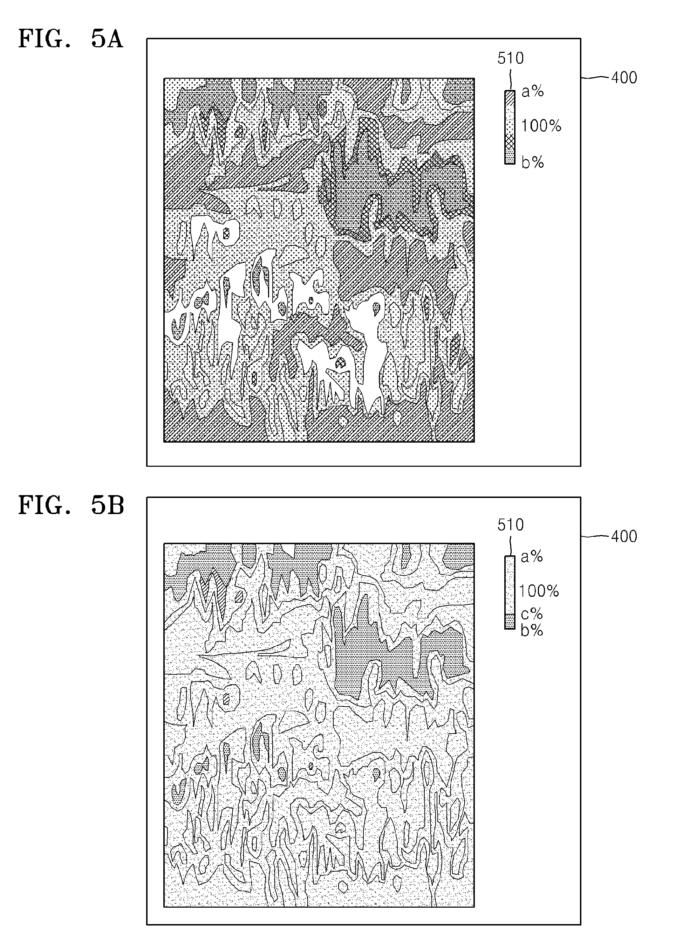

FIGS. 5A and 5B are diagrams illustrating an elasticity image according to an embodiment of the present invention.

The display 400 may map a ratio or a difference between a reference strain and a strain of each of a plurality of regions included in the elasticity image, to a color scale 510, and display a color corresponding to each of the plurality of regions in each of the plurality of regions. Each of the plurality of regions included in the elasticity image may include a predetermined number of pixel units.

In detail, the display 400 may acquire a color, which corresponds to a ratio or a difference between a reference strain and a strain of each of a plurality of regions included in the elasticity image, and display each of the plurality of regions in the corresponding color. The color scale 510 may include a plurality of colors corresponding to the ratio or the difference. In FIG. 5A, "b" of the color scale 510 may be a natural number that is greater than "a".

That is, even without receiving a selection of a reference region from the user, the display 400 may display a predetermined color in the elasticity image based on the reference strain that is determined by the strain determining unit 250.

As a ratio of the reference strain with respect to a strain of a predetermined region increases, the ratio may be mapped to a lower side of the color scale 510 illustrated in FIG. 5A. Also, when the ratio of the reference strain with respect to a strain of a predetermined region is great, the predetermined region may be harder than the reference similar tissue. Therefore, there is a high probability that the predetermined region will be a disease such as a malignant tumor.

The color scale 510 illustrated in FIG. 5A may include a lightness scale or a chroma scale of a first color.

The display 400 may display a region among the plurality of regions, a ratio or a difference between the reference strain and a strain of which is greater than a predetermined reference value, in a first color, and display a region among the plurality of regions, a ratio or a difference between the reference strain and a strain of which is smaller than or equal to the predetermined reference value, in a second color that is different from the first color. That is, referring to FIG. 5B, the display 400 may highlight a region having a high probability of being a disease by displaying a region among the plurality of regions, a ratio or a difference between the reference strain and a strain of which is greater than a predetermined reference value "c", in the first color, and displaying other regions in the second color.

The elasticity information providing unit 200 may further include a user input unit (not illustrated) that receives a selection of a reference region included in the elasticity image from the user. The user input unit may include a mouse, a keyboard, a track ball, a touchscreen, and the like, and may include various other input devices that are known to those of ordinary skill in the art.

The elasticity information providing unit 200 may change the reference strain based on a user input.

That is, when the user selects a region included in the elasticity image by using the user input unit, the strain determining unit 250 may change the reference strain based on a strain of the region selected by the user. The display 400 may map a ratio or a difference between the changed reference strain and a strain of each of a plurality of regions included in the elasticity image, to a color scale, and display a color corresponding to each of the plurality of regions in each of the plurality of regions. Accordingly, since the colors displayed in the elasticity image are changed based on the strain of the region of the elasticity image that is determined as a normal region by the user, a more accurate elasticity image may be displayed.

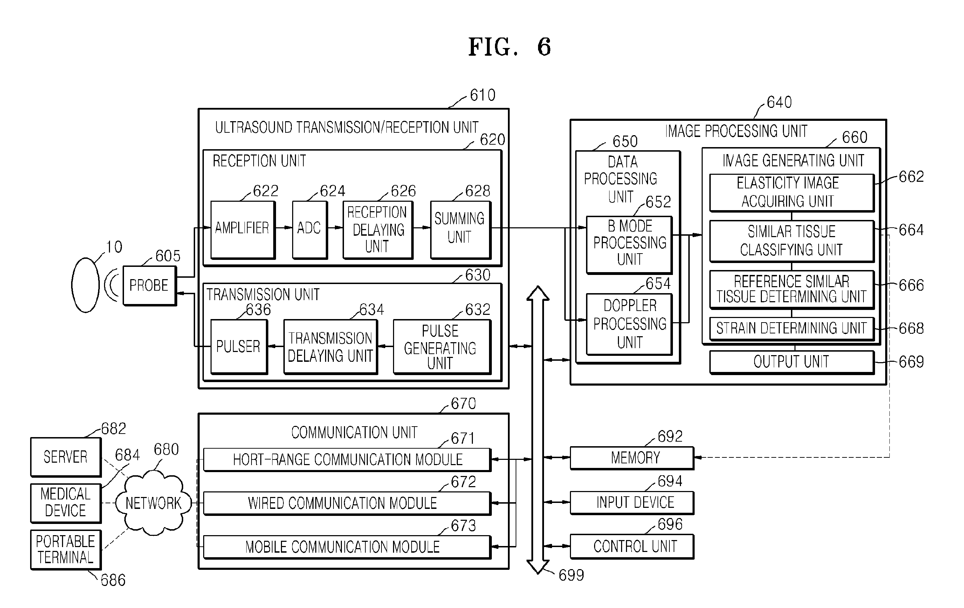

FIG. 6 is a block diagram illustrating an elasticity information providing apparatus 600 according to another embodiment of the present invention.

Referring to FIG. 6, the elasticity information providing apparatus 600 according to another embodiment of the present invention may include a probe 605, an ultrasound transmission/reception unit 610, an image processing unit 640, a communication unit 670, a memory 692, a user input unit 694, and a control unit 696, which may be connected to one another through a bus 699.

The elasticity information providing apparatus 600 may be embodied not only as a cart type device, but also as a portable device. Examples of the portable device may include a PACS viewer, a smart phone, a laptop computer, a personal digital assistant (PDA), and a tablet PC; however, embodiment of the present invention are not limited thereto.

The probe 605 transmits ultrasound waves to an object 10 based on a driving signal applied from the ultrasound transmission/reception unit 610 and receives echo signals reflected from the object 10. The probe 605 includes a plurality of transducers, and the plurality of transducers oscillate based on electric signals transmitted thereto and generate ultrasound waves, that is, acoustic energy. Also, the probe 605 may be connected to a main body of the elasticity information providing apparatus 600 wiredly or wirelessly. According to embodiments of the present invention, the elasticity information providing apparatus 600 may include a plurality of probes 605.

A transmission unit 630 supplies a driving signal to the probe 605 and includes a pulse generating unit 632, a transmission delaying unit 634, and a pulser 636. The pulse generating unit 632 generates pulses for forming transmission ultrasound waves based on a predetermined pulse repetition frequency (PRF), and the transmission delaying unit 634 applies a delay time for determining transmission directionality to the pulses. Pulses to which a delay time is applied correspond to a plurality of piezoelectric vibrators included in the probe 605, respectively. The pulser 636 applies a driving signal (or a driving pulse) to the probe 605 at a timing corresponding to each pulse to which a delay time is applied.

A reception unit 620 generates ultrasound data by processing echo signals received from the probe 605 and may include an amplifier 622, an analog-digital converter (ADC) 624, a reception delaying unit 626, and a summing unit 628. The amplifier 622 amplifies echo signals in each channel, and the ADC 624 analog-digital converts the amplified echo signals. The reception delaying unit 626 applies delay times for determining reception directionality to the digital-converted echo signals, and the summing unit 628 generates ultrasound data by summing the echo signals processed by the reception delaying unit 626.

The image processing unit 640 generates an ultrasound image by scan-converting ultrasound data generated by the ultrasound transmission/reception unit 610 and displays the ultrasound image. The ultrasound image may be not only a gray-scale ultrasound image obtained by scanning the object according to an amplitude (A) mode, a brightness (B) mode, and a motion (M) mode, but also a Doppler image of a motion of the object. The Doppler image may include a blood flow Doppler image (also referred to as a color Doppler image) representing a flow of blood, a tissue Doppler image representing a motion of a tissue, and a spectral Doppler image representing a movement speed of an object in a waveform.

A B mode processing unit 652 extracts B mode components from ultrasound data and processes the B mode components. An image generating unit 660 may generate an ultrasound image representing signal intensities as brightness based on the B mode components extracted by the B mode processing unit 652.

Likewise, a Doppler processing unit 654 may extract Doppler components from ultrasound data, and the image generating unit 660 may generate a Doppler image representing a motion of an object as colors or waveforms based on the extracted Doppler components.

The image generating unit 660 may generate a 3D ultrasound image through volume-rendering of volume data and may also generate an elasticity image that visualizes a deformation degree of the object 10 due to a pressure. In addition, the image generating unit 660 may display various additional information in an ultrasound image by using texts and graphics. The generated ultrasound image may be stored in the memory 692.

The image generating unit 660 may include an elasticity image acquiring unit 662, a similar tissue classifying unit 664, a reference similar tissue determining unit 666, and a strain determining unit 668. Since the elasticity image acquiring unit 662, the similar tissue classifying unit 664, the reference similar tissue determining unit 666, and the strain determining unit 668 have been described above, and thus detailed descriptions thereof will be omitted herein.

An output unit 669 displays the generated ultrasound image. The display unit 669 may display not only an ultrasound image, but also various information processed by the elasticity information providing apparatus 600 on a screen through a graphic user interface (GUI). The elasticity information providing apparatus 600 may include two or more output units 669 according to embodiments of the present invention. Also, the output unit 669 may output a ratio or a difference between the reference strain and a strain of an interesting region of the elasticity image that is input by the user.

The communication unit 670 is wiredly or wirelessly connected to a network 680 and communicates with an external device or a server. The communication unit 670 may exchange data with a hospital server or other medical apparatuses in a hospital connected through a Picture Archiving and Communication System (PACS). Also, the communication unit 670 may perform data communication according to the digital imaging and communications in medicine (DICOM) standard.

The communication unit 670 may transmit and receive data related to diagnosis of an object, such as an ultrasound image, ultrasound data, and Doppler data of the object, through the network 680 and may also transmit and receive medical images obtained by other medical devices, such as a CT image, a MRI image, and an X-ray image. In addition, the communication unit 670 may receive information related to diagnosis history or treatment schedule of a patient from a server and may utilize the information to diagnose the object 10. In addition, the communication unit 670 may perform data communication not only with a server or a medical device in a hospital, but also with a portable terminal of a doctor or a patient.

The communication unit 670 may be wiredly or wirelessly connected to the network 680 to exchange data with a server 682, a medical device 684, or a portable terminal 686. The communication unit 670 may include one or more components that enable communication with external devices, and may include, for example, a short-range communication module 672, a wired communication module 674, and a mobile communication module 676.

The short-range communication module 672 refers to a module for short-range communication within a predetermined distance. Examples of short-range communication techniques according to an embodiment of the present invention may include wireless LAN, Wi-Fi, Bluetooth, Zigbee, Wi-Fi Direct (WFD), ultra wideband (UWB), infrared data association (IrDA), Bluetooth Low Energy (BLE), and near field communication (NFC); however, embodiments of the present invention are not limited thereto.

The wired communication module 674 refers to a module for communication using electric signals or optical signals. Examples of wired communication techniques according to an embodiment of the present invention may include a pair cable, a coaxial cable, an optical fiber cable, and an Ethernet cable.

The mobile communication module 676 transmits and receives wireless signals with at least one of a station, an external terminal, and a server on a mobile communication network. Herein, the wireless signals may include voice call signals, video call signals, or various types of data for transmission and reception of text/multimedia messages.

The memory 692 stores various data processed by the elasticity information providing apparatus 600. For example, the memory 692 may store medical data related to diagnosis of the object, such as ultrasound data and ultrasound images that are input or output and may also store algorithms or programs to be executed in the elasticity information providing apparatus 600.

The memory 692 may be embodied as any of various storage media such as a flash memory, a hard disk drive, and an electrically erasable programmable read-only memory (EEPROM). Also, the elasticity information providing apparatus 600 may use a web storage or a cloud server that functions as the memory 692 on-line.

The user input unit 694 refers to a means through which the user inputs data for controlling the elasticity information providing apparatus 600. The user input unit 694 may include hardware components, such as a keypad, a mouse, a touch panel, a touchscreen, a track ball, and a jog switch. However, embodiments of the present invention are not limited thereto, and the user input unit 694 may further include various other input means such as an electrocardiogram measuring module, a respiration measuring module, a voice recognition sensor, a gesture recognition sensor, a fingerprint recognition sensor, an iris recognition sensor, a depth sensor, and a distance sensor.

The control unit 600 may control overall operations of the elasticity information providing apparatus 600. In other words, the control unit 696 may control operations among the probe 605, the ultrasound transmission/reception unit 610, the image processing unit 640, the communication unit 670, the memory 692, and the user input unit 694 illustrated in FIG. 6.

All or some of the probe 605, the ultrasound transmission/reception unit r100, the image processing unit 640, the communication unit 670, the memory 692, the user input unit 694, and the control unit 696 may be operated by software modules. However, embodiments of the present invention are not limited thereto, and some of the components described above may be operate by hardware modules. Also, at least one of the ultrasound transmission/reception unit 610, the image processing unit 640, and the communication unit 670 may be included in the control unit 696; however, embodiments of the present invention are not limited thereto.

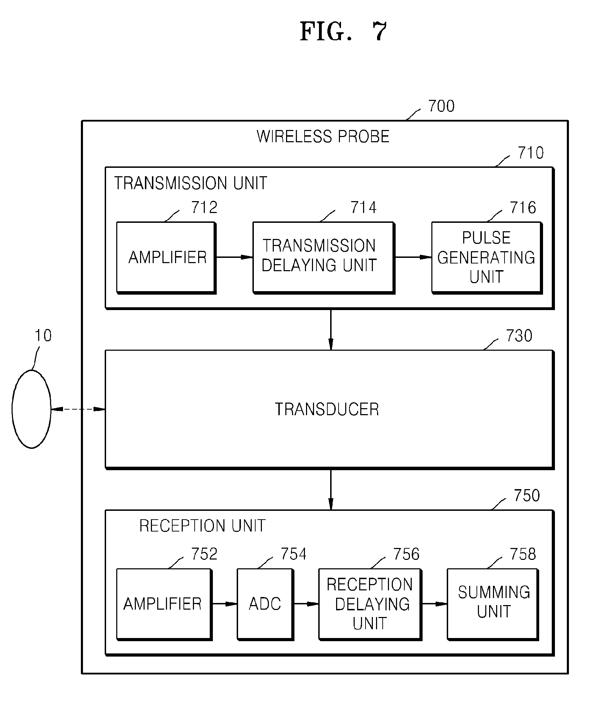

FIG. 7 is a block diagram illustrating a wireless probe 700 that may be connected to the elasticity information providing apparatus 600 according to another embodiment of the present invention. As described above with reference to FIG. 6, the wireless probe 700 may include a plurality of transducers, and, according to embodiments of the present invention, may include all or some of the ultrasound transmission/reception unit 610 illustrated in FIG. 6.

The wireless probe 700 according to the embodiment illustrated in FIG. 7 includes a transmission unit 710, a transducer 730, and a reception unit 750. Since descriptions thereof are given above with reference to FIG. 6, detailed descriptions thereof will be omitted. According to embodiments of the present invention, the wireless probe 700 may selectively include a reception delaying unit 765 and a summing unit 758.

The wireless probe 700 may transmit ultrasound signals to the object 10 and receive echo signals, and may generate ultrasound data and wirelessly transmit the ultrasound data to the elasticity information providing apparatus 600 illustrated in FIG. 6.

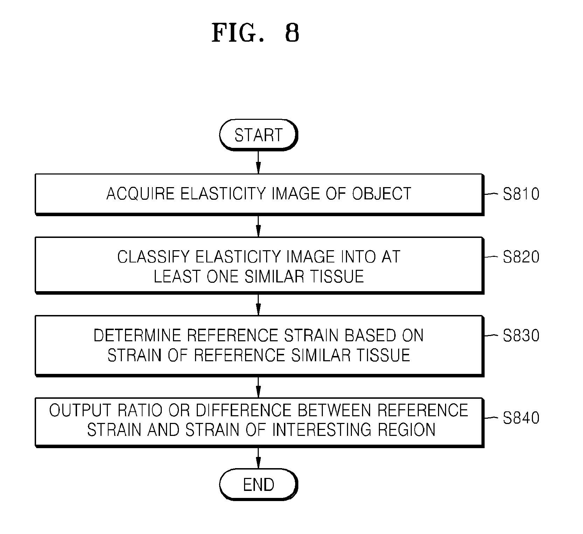

FIG. 8 is a flowchart illustrating an elasticity information providing method according to another embodiment of the present invention. Referring to FIG. 8, the elasticity information providing method according to another embodiment of the present invention may include operations that are sequentially performed by the elasticity information providing apparatus 200 illustrated in FIG. 2. Thus, even when there are omitted contents, the contents described above in relation to the elasticity information providing apparatus 200 illustrated in FIG. 2 may also be applied to the elasticity information providing method illustrated in FIG. 8.

In operation S810, the elasticity information providing apparatus 200 acquires an elasticity image of the object that is generated based on a strain between first ultrasound data of the object that is acquired while no pressure is applied to the object and second ultrasound data of the object that is acquired while pressure is applied to the object. The elasticity information providing apparatus 200 may acquire the elasticity image from an external server or an external medical apparatus, and may acquire the elasticity image of the object by photographing the elasticity image of the object.

In operation S820, the elasticity information providing apparatus 200 classifies the elasticity image into at least one similar tissue. The at least one similar tissue may include at least one of a skin tissue, a fat tissue, a mammary gland tissue, a muscle tissue, and a skeleton tissue of the object.

In operation S830, the elasticity information providing apparatus 200 determines a reference strain based on a stain of a reference similar tissue among the at least one similar tissue. The reference similar tissue may include a fat tissue of the object.

In operation S840, the elasticity information providing apparatus 200 provides the user with a ratio or a difference between the reference strain and a strain of an interesting region of the elasticity image that is input by the user.

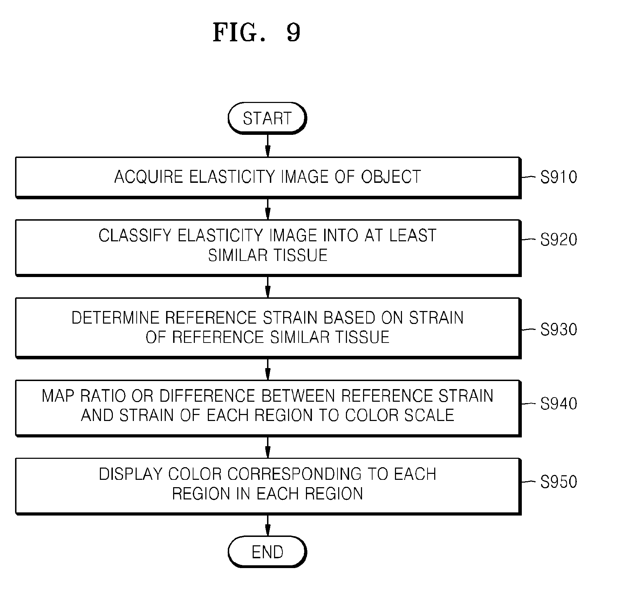

FIG. 9 is a flowchart illustrating an elasticity information providing method according to another embodiment of the present invention.

In operation S910, the elasticity information providing apparatus 200 acquires an elasticity image of the object that is generated based on a strain between first ultrasound data of the object that is acquired while no pressure is applied to the object and second ultrasound data of the object that is acquired while pressure is applied to the object.

In operation S920, the elasticity information providing apparatus 200 classifies the elasticity image into at least one similar tissue.

In operation S930, the elasticity information providing apparatus 200 determines a reference strain based on a stain of a reference similar tissue among the at least one similar tissue.

In operation S940, the elasticity information providing apparatus 200 maps a ratio or a difference between the reference strain and a strain of each of a plurality of regions included in the elasticity image to a color scale.

In operation S950, the elasticity information providing apparatus 200 displays a color corresponding to each of the plurality of regions in each of the plurality of regions.

The embodiments of the present invention may be written as computer programs and may be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs, DVDs, etc.), and transmission media such as Internet transmission media.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. An apparatus for providing elasticity information, comprising:
an elasticity image acquiring unit configured to acquire an elasticity image of an object;
a similar tissue classifying unit configured to classify the elasticity image into at least one similar tissue;
a strain determining unit configured to determine a reference strain based on a strain of a reference similar tissue among the at least one similar tissue; and
an output unit configured to output a ratio or a difference between the reference strain and a strain of an interesting region of the elasticity image.

2. The apparatus of claim 1, wherein the output unit maps a ratio or a difference between the reference strain and a strain of each of a plurality of regions comprised in the elasticity image, to a color scale, and displays a color corresponding to each of the plurality of regions in each of the plurality of regions.

3. The apparatus of claim 2, wherein the color scale comprises a lightness scale or a chroma scale of a first color.

4. The apparatus of claim 2, wherein the display displays a region among the plurality of regions, a ratio or a difference between the reference strain and a strain of which is greater than a predetermined reference value, in a first color, and displays a region among the plurality of regions, a ratio or a difference between the reference strain and a strain of which is smaller than or equal to the predetermined reference value, in a second color that is different from the first color.

5. The apparatus of claim 2, further comprising a user input unit configured to receive a selection of a region comprised in the elasticity image, from the user,
wherein
the strain determining unit changes the reference strain according to a strain of the region selected by the user, and
the display maps a ratio or a difference between the reference strain and a strain of each of a plurality of regions comprised in the elasticity image, to a color scale, and displays a color corresponding to each of the plurality of regions in each of the plurality of regions.

6. The apparatus of claim 1, wherein the similar tissue classifying unit classifies the elasticity image into at least one similar tissue based on at least one of an attenuation coefficient value, a phase value, and a intensity value of each of a plurality of first response signals that are reflected from the object while pressure is not applied to the object.

7. The apparatus of claim 1, wherein the similar tissue classifying unit classifies the elasticity image into at least one similar tissue based on at least one of an attenuation coefficient value, a phase value, and a intensity value of each of a plurality of second response signals that are reflected from the object while pressure is applied to the object.

8. The apparatus of claim 1, wherein the similar tissue classifying unit classifies the elasticity image into at least one similar tissue based on at least one of a texture of the elasticity image, a geometry of the elasticity image, and a gradient of the elasticity image.

9. The apparatus of claim 1, wherein the elasticity image of the object is generated based on a strain between first ultrasound data of the object, which is acquired while pressure is not applied to the object, and second ultrasound data of the object, which is acquired while pressure is applied to the object, and wherein the similar tissue classifying unit classifies a brightness (B) mode image of the object, which is generated by using the first ultrasound data or the second ultrasound data, into at least one similar tissue, and classifies the elasticity image into at least one similar tissue based on location information of each of the at least one similar tissue of the B mode image.

10. The apparatus of claim 1, further comprising a reference similar tissue determining unit configured to determine a region, which emits a first response signal having at least one of an attenuation coefficient value within a predetermined attenuation coefficient range, a phase value within a predetermined phase range, and a intensity value within a predetermined intensity range among a plurality of first response signals that are reflected from the object while pressure is not applied to the object, as the reference similar tissue.

11. The apparatus of claim 1, wherein further comprising a reference similar tissue determining unit configured to determine a region, which emits a second response signal having at least one of an attenuation coefficient value within a predetermined attenuation coefficient range, a phase value within a predetermined phase range, and a intensity value within a predetermined intensity range among a plurality of second response signals that are reflected from the object while pressure is applied to the object, as the reference similar tissue.

12. An apparatus for providing elasticity information, comprising:
an elasticity image acquiring unit configured to acquire an elasticity image of an object;
a similar tissue classifying unit configured to classify the elasticity image into at least one similar tissue;
a strain determining unit configured to determine a reference strain based on a strain of a reference similar tissue among the at least one similar tissue; and
an output unit configured to map a ratio or a difference between the reference strain and a strain of each of a plurality of regions comprised in the elasticity image, to a color scale, and display a color corresponding to each of the plurality of regions in each of the plurality of regions.

13. A method for providing elasticity information, comprising:
acquiring an elasticity image of an object;
classifying the elasticity image into at least one similar tissue;
determining a reference strain based on a strain of a reference similar tissue among the at least one similar tissue; and
outputting a ratio or a difference between the reference strain and a strain of an interesting region of the elasticity image.

14. A method for providing elasticity information, comprising:
acquiring an elasticity image of an object;
classifying the elasticity image into at least one similar tissue;
determining a reference strain based on a strain of a reference similar tissue among the at least one similar tissue; and
mapping a ratio or a difference between the reference strain and a strain of each of a plurality of regions comprised in the elasticity image, to a color scale, and displaying a color corresponding to each of the plurality of regions in each of the plurality of regions.

15. A non-transitory computer-readable recording medium that stores a program that, when executed by a computer, performs the method of any one of claims 13 to 14.
